# EUROPEAN PATENT SPECIFICATION

(11) **EP 0 406 660 B1**
(45) Date of publication and mention of the grant of the patent: **13.10.1993**
(21) Application number: 90112005.5
(22) Date of filing: 25.06.1990
(51) Int. Cl.: C07C 69/63, C07C 67/11

(54) **A process for producing haloalkyl alkanoates**
Verfahren zur Herstellung von Haloalkylalkanoaten
Procédé pour la préparation de haloalcoylalcanoates

(30) Priority: 07.07.1989 JP 176251/89
(43) Date of publication of application: 09.01.1991
(73) Proprietor: SHIONOGI SEIYAKU KABUSHIKI KAISHA, Osaka 541 (JP)
(72) Inventor: Tanaka, Mamoru, Ibaraki-shi, Osaka (JP); Hajima, Makoto, Hirakata-shi, Osaka (JP); Hamashima, Yoshio, Kyoto-shi, Kyoto (JP)
(74) Representative: Kügele, Bernhard

(56) References cited:
- DE-C- 362 747
- US-A- 2 500 009
- CHEMICAL ABSTRACTS, vol. 104, no. 13, March 31, 1986 Columbus, Ohio, USA G. WEINGARTEN "Cephalosporin antibiotics" page 697, column 1, abstract-no. 109 344g; & JP-A-60 172 989
- CHEMICAL ABSTRACTS, vol. 101, no. 21, November 19, 1984 Columbus, Ohio, USA V.J. JASYS et a. "1,1-Al- kanediol dicarboxylate-linked antibacterial agents" page 733, column 2, abstract-no. 191 536r; & JP-A-58 116 486

## Description

This invention relates to a process for producing alkanoic acid halogenated alkyl ester useful as a reagent for preparing some oral antibacterials. More specifically, it relates to a process comprising a reaction of alkanoic acid salt (I) with an haloalkane compound containing two reactive halogen atoms to economically and safely produce haloalkyl alkanoate (III).

An industrial process for preparing haloalkyl alkanoate (III) by reacting alkanal and acid chloride gives toxic by-products. The inventors sought a safer and economical method without producing such by-products and found the process of this invention. According to this invention, haloalkyl alkanoate (III) is produced by treating alkanoic acid salt (I) and 10 equivalents or more of a halogenated alkane compound (II) containing two reactive halogen atoms, in 2 to 5 parts by weight of alkanoic acid dialkylamide. 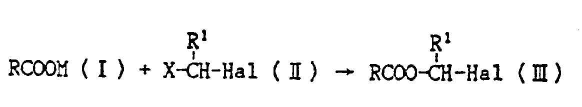
wherein R and R¹ each is hydrogen or chlorine or an optionally substituted alkyl, M is a salt forming atom or group, and X and Hal each is halogen, during a reaction time of at least 30 hours.

The reaction temperature is usually -30 to 100C° (preferably 50° or lower; especially 0 to 30°C) and the reaction time is 30 hours or longer, usually up to 200 hours, especially 40 to 100 hours.
The reaction may be carried out in a medium in the absence or presence of a subreagent (e.g., inert solvent, lithium halide, lithium carbonate, phase transfer reagent, crown ether, alkali metal halide, alcohol, water).

Alkanoic acid salt (I) and haloalkane (II), thus, haloalkyl alkanoate (III) may have an inert substituent as given below.

The alkanoic acid of alkanoic acid salt (I) can be 2C to 11C straight, branched, or cyclic alkanoic acid. R is preferably 1C to 10C straight, branched or cyclic alkyl, especially, methyl, ethyl, propyl, isopropyl, butyl, isobutyl or tert-butyl. More preferably, the alkanoic acid is acetic acid, propionic acid, isobutanoic acid, pivalic acid, cyclohexanecarboxylic acid, cyclopentaneacetic acid, cyclohexaneacetic acid, octanoic acid. Especially preferable are acetic acid, pivaloic acid and cyclohexaneacetic acid.

Preferably, alkanoic acid salt (I) can be a light metal (e.g., alkali metal, alkaline earth metal, or the like atom of Group I to III. Period 2 to 3) salt or quaternary ammonium salt. M is preferably a light metal atom or quaternary ammonium group. Better M is an alkali metal (e.g., sodium, potassium) atom or an alkaline earth metal (e.g., magnesium, calcium) atom. Primary to tertiary organic amine salts could not be used efficiently. Alkanoic acid salt (I) may be made prior to or during the reaction from a base and alkanoic acid in situ.

In the haloalkane compound (II) R¹ is preferably hydrogen or lower alkyl (i.e. 1C to 8C alkyl) and Hal and/or X is preferably chlorine or bromine.

Representative as haloalkane (II) are dichloromethane, chlorobromomethane, chloroiodomethane, dibromomethane, bromoiodomethane, chloroform, dichloroethane, trichloroethane, chlorobromoethane, chlorodibromoethane, chloroiodoethane, dichlorobromoethane, dichloroiodoethane, dibromoethane, bromoiodoethane, chlorodiiodoethane, chlorobromopropane, chloroiodopropane, dibromopropane, bromoiodopropane, diiodochloropropane. Excess haloalkane (II) can be recovered from the reaction mixture.

Representative inert substituent for R or R¹ in compounds (I), (II), and (III) include carbon function (e.g., straight, branched, or cyclic alkyl, alkenyl, alkynyl, aralkyl, aryl, heterocyclyl, carboxylic acyl, carbamoyl, protected carboxy, cyano); nitrogen function (e.g., acylamino, alkylamino, dialkylamino, isocyano, nitro); oxygen function (e.g., alkoxy, aryloxy, heterocyclyloxy, cyanato, carboxylic acyloxy, sulfonic acyloxy, phosphoric acyloxy); sulfur function (e.g., alkylthio, alkylsulfonyl, arylthio, arylsulfonyl, heterocyclylthio, heterocyclylsulfonyl, acylthio, thioxo): halo (e.g., fluoro, chloro); silyl (e.g., trialkylsilyl, dialkylalkoxysilyl).

Known methods for producing haloalkyl alkanoate (III) include, for example, those for chloroalkyl ester, e.g., the method of Journal of American Chemical Society, Volume 53, page 660 (1921) by reacting alkanoic acid chloride and paraformaldehyde ; and the method of Journal of American Chemical Society, Volume 89, page 5439 (1967) by reacting pivaloic acid chloride, formaldehyde and zinc chloride and those for alkanoic acid iodoalkyl ester of German Patent Application Publication (OLS) No. 1931012 by reacting said chloroalkyl alkanoate with alkali metal iodide. All these known methods give toxic by-products.

The effects of this invention over closely related known methods for aliphatic esters can be illustrated as follows :

### (1) Japanese Patent Application Kokai No. 46-2821 :

The reaction of alkali metal salt or trialkylamine salt of various aminoacids and chloroiodomethane (0.9 to 1.5 volume) in N,N-dimethylformamide (abbreviated as DMF : 2.3 to 10 volume) at room temperature for 1.5 hours to overnight giving chloromethyl ester. In the case of alkanoic acid salt (I), the yield of objective product (III) reached to practical when the reaction time was 30 hours or more and amount of dihaloalkane (II) was 10 equivalents or more. When trialkylamine salt was used, the objective product (III) could not be found although much chloromethyltriethylammonium bromide and methylenebis ester were obtained.

### (2) Journal of the Medicinal Chemistry, Volume 22, Page 658 (1979) :

The method for preparing chloromethyl ester by reacting N-protected various amino acids with triethylamine (1.7 equivalent) in DMF (11.4 volumes) to make salt and then treating the salt with chloroiodomethane (ca. 4 equivalents) for 19 hours to give chloromethyl ester in 8.7 to 25 % yield. In the case of alkanoic acid salt (I) of this invention, the yield of objective product (III) reached to practical when the reaction time was 30 hours or more and the yield and selectivity of the objective product (III) were high only when the amount of dihaloalkane (II) was 10 equivalents or more. When trialkylamine salt was used, the objective product (III) could not be found although much chloromethyltriethylammonium bromide and methylenebis ester were obtained.

### (3) Japanese Patent Application Kokai No. 58-116486 :

The method for preparing chloromethyl ester by reacting penicillin tetrabutylammonium salt with 0.1 to 10 equivalents (especially 10 to 23 equivalents) of dihalomethane (especially bromochloromethane, chloroiodomethane) at 0 to 80 °C (especially room temperature) for 10 minutes to 24 hours (especially overnight), if required in a solvent as DMF (especially without any solvent). In the case of alkanoic acid salt (I) of this invention, the yield of objective product (III) reached to practical when the reaction time is 30 hours or more and no objective product was found in the absence of amide (especially DMF).

### (4) Japanese Patent Application Kokai No. 60-172989 :

The method for preparing a 2 : 3 mixture of chloromethyl ester and methylenebis ester by reacting alkoxyalkanoic acid (especially methoxyisobutanoic acid) and potassium carbonate in DMF (20 volumes) to make salt and treating it with dihaloalkane (especially chloroiodomethane, especially 1.8 equivalents) and base (especially sodium carbonate) at room temperature for 2.5 hours. In the case of alkanoic acid salt (I) of this invention, the yield of objective product (III) reached to practical when the reaction time was 30 hours or more and the amount of dihaloalkane (II) was 10 equivalents or more.

Conventional conditions (e.g., solvent, stirring, dry condition, inert gas) are applicable for the reaction of this invention.

Preferably, the reaction solvents are, for example, hydrocarbons (e.g., pentane, hexane, octane, benzene, toluene, xylene), halogenated hydrocarbons (e.g., dichloromethane, chlorobromomethane, chloroiodomethane, dibromomethane, bromoiodomethane, dichloroethane, chlorobromo ethane, chloroiodoethane, dibromoethane, bromoiodoethane, trichloroethane, dichlorobromoethane, chlorodibromoethane, dichloroiodoethane, chlorodiiodoethane, chlorobromopropane, chloroiodopropane, dibromopropane, bromoiodopropane, diiodochloropropane, carbon tetrachloride, dichloroethane, chloroform, trichloroethane, chlorobenzene), ether (e.g., diethyl ether, methyl isobutyl ether, dioxane, tetrahydrofuran) ketone (e.g., acetone, methyl ethyl ketone, cyclohexanone), ester (e.g,, ethyl acetate, isobutyl acetate, methyl benzoate), nitrohydrocarbon (e.g., nitromethane, nitrobenzene), nitrile (e.g., acetonitrile benzonitrile), amide (e.g., dimethylformamide, dimethylacetamide, acetylmorpholine, hexamethylphosphorotriamide), sulfoxide (e.g., dimethyl sulfoxide), organic base (e.g., diethylamine, triethylamine, pyridine, picoline, collidine, quinoline), water, or other industrial solvents or mixtures of these. Aprotic solvents are more preferable among these.

Haloalkyl alkanoate (III) includes known reagents for introducing so-called physiologically active ester groups to an injectable betalactam antibacterial in order to make the ester orally available. For example, such oral beta-lactam antibacterials include pivampicillin, pivmecillinam, cefteram pivoxyl, and the like.

Objective haloalkyl alkanoate (III) can be recovered from the reaction mixture by removing contaminants (e.g., by-products, unreacted starting materials, solvent) by conventional methods (e.g., extraction, evaporation, washing, concentration, precipitation, filtration, drying) and then isolating by conventional work up (e.g., absorption, elimination, distillation, chromatography).

Following Examples illustrate the embodiment of this invention.

### Example 1 (Acetoxymethyl)

To a stirred mixture of potassium acetate 4.2 g (42 millimole) in DMF (21 g: 5.0 parts by weight) is added bromoiodomethane (97 g: 10.5 equivalents) and the mixture is kept at room temperature for 50 hours. The reaction mixture is washed with water, dried, and distilled at atmospheric pressure to give bromomethyl acetate (2.8 g: bp₁₄₀ 86°C: Yield 44 %).
NMR(CDCl₃) : 2.12 (s, 3H), 5.77 (s, 2H).

### Example 2 (Acetoxyethyl)

To a stirred mixture of DMF (30 g: 3.1 parts by weight) and potassium acetate (9.8 g: 0.1 moles) is added 1-bromo-1-chloroethane (30 g: 21 equivalents). The mixture is kept at room temperature for 24 hours and at 50°C for 8 hours. The reaction mixture is washed with water, dried, and distilled at atmospheric pressure to collect the part boiling at 110 to 130°C to give 1-chloroethyl acetate (2.4 g: Yield 20 %).
NMR (CDCl₃): 1.95 (d, J=7Hz, 3H), 2.08 (s, 3H), 6.67 (q, J=7Hz, 1H).

### Example 3 (Pivaloyloxymethyl)

To a stirred mixture of DMF (28.4 g: 2.8 parts by weight) and potassium pivalate (10 g: 71 millimole) is added bromochloromethane (184.5 g: 20 equivalents) and the mixture is kept at room temperature for 72 hours. The reaction mixture is washed with water, dried, and distilled at atmospheric pressure to collect the part boiling at 146 to 148°C to give chloromethyl pivalate (6.26 g: Yield 59 %) and dipivaloyloxymethane (1.14 g: Yield 15 %).
NMR (CDCl₃) : 1.25 (s, 9H), 5.72 (s, 2H).

### Example 4 (Pivaloyloxymethyl)

To a stirred mixture of DMF (7.56 g: 2.1 parts by weight) and potassium pivalate (3.6 g: 26 millimole) is added bromochloromethane (99 g: 30 equivalents) and the mixture is kept at room temperature for 96 hours. The reaction mixture is washed with water, dried, and distilled at atmospheric pressure to give chloromethyl pivalate (1.74 g: Yield 45 %) and dipivaloyloxymethane (0.57 g: Yield 21 %).
NMR (CDCl₃) : 1.25 (s, 9H), 5.72 (s, 2H).

### Example 5 (Pivaloyloxymethyl)

To a stirred mixture of DMF (2.3 g: 2 parts by weight) and potassium pivalate (1.2 g: 9 millimole) is added bromochloromethane (55 g: 50 equivalents) and the mixture is kept at 0°C for 40 hours. The reaction mixture is washed with water, dried, and distilled at atmospheric pressure to give chloromethyl pivalate (0.3 g: Yield 23 %) and dipivaloyloxymethane (0.13 g: Yield 14 %).
NMR (CDCl₃) : 1.25 (s,9H), 5.72 (s,2H).

### Example 6 (Pivaloyloxymethyl)

To a stirred mixture of DMF (12 g: 3.7 parts by weight) and sodium pivalate (3.25 g: 26 millimole) is added bromochloromethane (78 g: 23 equivalents) and the mixture is kept at room temperature for 72 hours. The reaction mixture is washed with water, dried, and distilled at atmospheric pressure to collect the part boiling at 148°C to give chloromethyl pivalate (1.89 g: Yield 55 %).
NMR (CDCl₃) : 1.25 (s, 9H), 5.72 (s, 2H).

### Example 7 (Pivaloyloxyethyl)

To a stirred mixture of DMF (12 g: 3.2 parts by weight) and dry sodium pivalate (3.7 g: 30 millimole) is added 1-bromo-1-chloroethane (90 g: 21 equivalents) and the mixture is kept at room temperature for 24 hours and at 50°C for 8 hours. The reaction mixture is washed with water, dried, and distilled at atmospheric pressure to give 1-chloroethyl pivalate (0.8 g: Yield 16 %).
NMR (CDCl₃): 1.23 (s, 9H), 1.78 (d, J=7Hz, 3H), 6.53 (q, J=7Hz, 1H).

### Example 8 (Isobutyryloxymethyl)

To a stirred mixture of DMF (12 g: 3.1 parts by weight) and potassium isobutyrate (3.85 g: 30 millimole) is added bromochloromethane (78 g: 20 equivalents) and the mixture is kept at room temperature for 60 hours and at 50°C for 8 hours. The reaction mixture is washed with water, dried, and distilled at atmospheric pressure to collect the part boiling at 135 to 140°C to give chloromethyl 2-methylpropionate (1.4 g: Yield 39 %).
NMR (CDCl₃): 1.23 (s, 9H), 1.78 (d, J=7Hz, 3H), 6.53 (q, J=7Hz, 1H).

### Example 9 (Propionyloxymethyl)

To a stirred mixture of DMF (12 g: 3.5 parts by weight) and potassium propionate (34 g: 30 millimole) is added bromochloromethane (78 g: 20 equivalents) and the mixture is kept at room temperature for 24 hours and at 50°C for 8 hours. The reaction mixture is washed with water, dried, and distilled at atmospheric pressure to give chloromethyl propionate (0.8 g: bp. 133°C: Yield 22%).
NMR (CDCl₃) 1.23 (s, 9H), 1.78 (d, J=7Hz, 3H), 6.53 (q, J=7Hz, 1H).

### Example 10 (Propionyloxymethyl)

To a stirred mixture of DMF (32 g 3.5 parts by weight) and potassium propionate (9.2 g: 81 millimole) is added bromochloromethane (210 g: 20 equivalents) and the mixture is kept at 30°C for 50 hours. The reaction mixture is washed with water, dried, and distilled at atmospheric pressure to give propionic acid chloromethyl ester (75 g: bp. 133°C: Yield 28 %).
NMR (CDCl₃): 1.23 (s, 9H), 1.78 (d, J=7Hz, 3H), 6.53 (q J=7Hz, 1H).

## Claims

1. A process for producing haloalkyl alkanoate (III) which comprises treating alkanoic acid salt (I) with 10 equivalents or more of a halogenated alkane compound (II) containing two reactive halogen atoms, in 2 to 5 parts by weight of alkanoic acid dialkylamide. 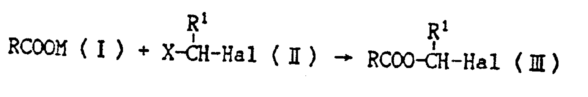 wherein R and R¹ each is hydrogen or chlorine, or an optionally substituted alkyl, M is a salt forming atom or group, and X and Hal each is halogen, during a reaction time of at least 30 hours.

2. A process as claimed in Claim 1 wherein R is 1C to 10C straight, branched or cyclic alkyl.

3. A process as claimed in Claim 1 wherein R is methyl, ethyl, propyl, isopropyl, butyl, isobutyl or tert-butyl.

4. A process as claimed in Claim 1 wherein M is a metal of Groups 1a, 2a or Group 3a, period 2 or 3, of the Periodic Table of the elements or a quaternary ammonium.

5. A process as claimed in Claim 1 wherein M is an alkali metal or alkaline earth metal atom.

6. A process as claimed in Claim 1 wherein R¹ is hydrogen or 1C to 8C alkyl and X and Hal each is chlorine or bromine.

7. A process as claimed in Claim 1 wherein the reaction temperature is 50°C or lower.

## Patentansprüche

1. Verfahren zur Herstellung von Haloalkylalkanoat (III), das die Behandlung eines Alkansäuresalzes (I) mit 10 Äquivalenten oder mehr einer halogenierten Alkanverbindung (II) mit zwei reaktiven Halogenatomen in 2 bis 5 Gewichtsteilen Alkansäure-Dialkylamid 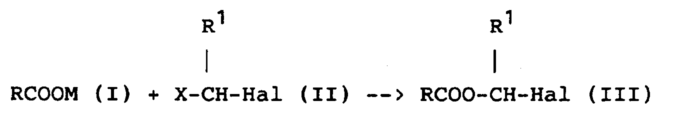 umfaßt, worin R und R¹ jeweils Wasserstoff oder Chlor oder ein, gegebenenfalls substituiertes, Alkyl ist, M ein salzbildendes Atom oder eine solche Gruppe ist und X und Hal jeweils Halogen sind, während einer Reaktionszeit von zumindest 30 Stunden.

2. Verfahren nach Anspruch 1, bei dem R eine gerade, verzweigte oder zyklische Alkylgruppe mit 1C bis 10C ist.

3. Verfahren nach Anspruch 1, bei dem R eine Methyl-, Äthyl-, Propyl-, Isopropyl-, Butyl-, Isobutyl- oder tert.-Butylgruppe ist.

4. Verfahren nach Anspruch 1, bei dem M ein Metall der Gruppen 1a, 2a oder der Gruppe 3a der Perioden 2 oder 3 des Periodischen Systems der Elemente oder ein quaternäres Ammonium ist.

5. Verfahren nach Anspruch 1, bei dem M ein Alkalimetall- oder Erdalkalimetallatom ist.

6. Verfahren nach Anspruch 1, bei dem R¹ Wasserstoff oder eine Alkylgruppe mit 1C bis 8C und X und Hal jeweils Chlor oder Brom sind.

7. Verfahren nach Anspruch 1, bei dem die Reaktionstemperatur 50°C beträgt oder niedriger ist.

## Revendications

1. Procédé de préparation d'un alcanoate d'haloalkyle (III) qui comprend le traitement d'un sel d'acide alcanoïque (I) par 10 équivalents ou davantage d'un alcane halogéné (II) contenant deux atomes d'halogène réactifs, dans 2 à 5 parties en poids d'un dialkylamide d'acide alcanoïque. 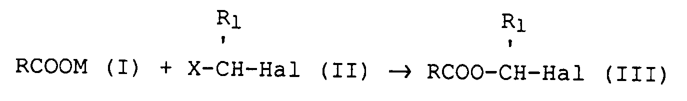 Dans ce schéma réactionnel, R et R¹ sont chacun un atome d'hydrogène ou de chlore, ou un groupe alkyle éventuellement substitué, M est un atome ou groupe formant un sel, et X et Hal sont chacun un atome d'halogène, pendant une durée de réaction d'au moins 30 heures.

2. Procédé selon la revendication 1, dans lequel R est un groupe alkyle en C₁ à C₁₀ linéaire, ramifié ou cyclique.

3. Procédé selon la revendication 1, dans lequel R est un groupe méthyle, éthyle, propyle, isopropyle, butyle, isobutyle ou tert-butyle.

4. Procédé selon la revendication 1, dans lequel M est un métal des groupes 1a, 2a ou du Groupe 3a, périodes 2 ou 3, de la Classification Périodique des Eléments ou un ammonium quaternaire.

5. Procédé selon la revendication 1, dans lequel M est un atome de métal alcalin ou alcalino-terreux.

6. Procédé selon la revendication 1, dans lequel R¹ est un atome d'hydrogène ou un groupe alkyle en C₁ à C₈ et X et Hal sont chacun un atome de chlore ou de brome.

7. Procédé selon la revendication 1, dans lequel la température de réaction est de 50°C ou moins.
